# EUROPÄISCHE PATENTSCHRIFT

(11) **EP 1 663 019 B1**
(45) Veröffentlichungstag und Bekanntmachung des Hinweises auf die Patenterteilung: **13.02.2008**
(21) Anmeldenummer: 03757835.8
(22) Anmeldetag: 13.09.2003
(51) Int. Cl.: A61B 17/15, A61B 19/00

(54) **VORRICHTUNG ZUR BESTIMMUNG DES WINKELS ZWISCHEN FEMUR UND TIBIA**
DEVICE FOR DETERMINING THE ANGLE BETWEEN THE FEMUR AND THE TIBIA
DISPOSITIF POUR DETERMINER L'ANGLE ENTRE LE FEMUR ET LE TIBIA

(43) Veröffentlichungstag der Anmeldung: 07.06.2006
(73) Patentinhaber: AESCULAP AG & Co. KG, 78532 Tuttlingen (DE)
(72) Erfinder: LEITNER, François, F-38410 Uriage (FR); LAUGIER, Yann, F-38610 Jieres (FR)
(74) Vertreter: Hoeger, Stellrecht & Partner Patentanwälte
(86) Internationale Anmeldenummer: PCT/EP2003/010215
(87) Internationale Veröffentlichungsnummer: WO 2005/032382

(56) Entgegenhaltungen:
- WO-A-02/09596
- WO-A-02/36031
- WO-A-02/067800
- DE-U- 20 217 014
- US-A1- 2002 055 679
- US-B1- 6 554 837

## Beschreibung

Die Erfindung betrifft eine Vorrichtung zur Bestimmung des Winkels zwischen Femur und Tibia bei der Implantation einer unikondylären Knieprothese mit einem Navigationssystem und einer Datenverarbeitungseinrichtung.

In der WO 02/36031 A1 wird eine Vorrichtung beschrieben, bei der eine Datenverarbeitungsanlage aufgrund der geometrischen Daten des einzusetzenden Implantates und aufgrund einer angenommenen Anlagefläche für das Implantat geometrische Daten des Kniegelenkes nach der Implantation berechnet. Es handelt sich dabei um eine Totalendoprothese, also ein Implantat, durch das das gesamte Kniegelenk mit seinen Gelenkflächen ersetzt wird. Das bekannte Implantat soll so eingesetzt werden, dass dessen Gelenkfläche senkrecht auf einer Achse steht, die durch den Kniemittelpunkt und durch den Knöchel bzw. das Hüftgelenk hindurchgeht, außerdem soll das Implantat mit der höchsten Stelle der ursprünglichen Gelenkfläche übereinstimmen. Damit ist die Lage des Implantates im Kniegelenk vorgegeben, und dies gilt auch für die Winkel zwischen Tibia und Femur, die bei dieser Implantation immer gleich sind und allein durch die Achsen von Femur und Tibia vor der Implantation bestimmt werden. Ein Vorgehen bei unikondylären Knieprothesen wird in dieser Druckschrift nicht beschrieben.

Auch in der DE 202 17 014 U1 wird eine Totalendoprothese des Knies beschrieben, also eine Prothese, die alle Gelenkflächen ersetzt. Mit einer Datenverarbeitungseinrichtung können unterschiedliche Winkellagen des Implantates relativ zu Tibia und/oder Femur bestimmt werden und die aus dieser Verschwenkung resultierenden Winkel zwischen Tibia und Femur. Diese unterschiedlichen Winkellagen ergeben sich allein durch die Winkelstellung des Implantates. Im Rahmen dieser Druckschrift werden nur Totalendoprothesen erörtert, das Problem der unikondylären Implantate wird nicht angesprochen.

Neben dem Ersatz eines natürlichen Kniegelenks durch eine vollständige Knieendoprothese ist es in einigen Fällen wünschenswert, das natürliche Kniegelenk nur einseitig zu ersetzen, also nur eine der beiden Kondylen des Kniegelenkes mittels einer Endoprothese zu ersetzen, die andere Gelenkfläche jedoch unverändert zu lassen. Beim Implantieren derartiger unikondylärer Endoprothesen wird sowohl auf den Femur als auch auf die Tibia jeweils ein unikondyläres Implantat aufgesetzt, und diese beiden Implantate ersetzen gegebenenfalls unter Zwischenlage eines Gleitkörpers die natürlichen Gelenkflächen von Femur und Tibia auf einer Seite. Zur Implantation dieser unikondylären Implantate müssen sowohl Tibia als auch Femur bearbeitet werden, die natürlichen Gelenkflächen müssen entfernt werden und es müssen Anlageflächen für diese unikondylären Implantate in den Knochen eingearbeitet werden. Die Lage dieser Anlageflächen muss entsprechend den Abmessungen der verwendeten unikondylären Implantate gewählt werden.

Es hat sich nun herausgestellt, dass die Lage dieser Anlageflächen und damit die Lage der unikondylären Implantate den Winkel zwischen der Längsachse des Femurs und der Längsachse der Tibia sehr empfindlich beeinflusst. Dies gilt sowohl hinsichtlich einer Translation des Implantates parallel zur Längsrichtung des Femurs bzw. der Tibia als auch hinsichtlich einer Verschwenkung des Implantates gegenüber einer Ebene, die senkrecht auf der Längsachse von Tibia bzw. Femur steht.

Es ist Aufgabe der Erfindung, eine Vorrichtung der eingangs beschriebenen Art so auszubilden, dass mit ihr der Winkel zwischen Femur und Tibia in Abhängigkeit von der Implantationslage der unikondylären Implantate bestimmt werden kann.

Diese Aufgabe wird bei einer Vorrichtung der eingangs beschriebenen Art erfindungsgemäß durch eine Ausgestaltung gemäß Anspruch 1 gelöst.

Die Vorrichtung berechnet also aus den Anlageflächen für das unikondyläre Implantat und damit aufgrund der Lagedaten, also der Position im Raum und der Orientierung im Raum, und aufgrund der Abmessungen des gewünschten Implantates zusammen mit den Lagedaten der unveränderten Gelenkfläche die Gesamtgeometrie des Knies. Aus dieser Gesamtgeometrie ergibt sich dann auch der Winkel zwischen der Längsachse der Tibia und der Längsachse des Femurs. Durch Veränderung der Lagedaten der Anlagefläche lässt sich dieser Winkel beeinflussen, so dass der Operateur die Möglichkeit hat, Winkelkorrekturen vorzunehmen.

All diese Vorgänge werden durchgeführt, bevor die Anlageflächen in den Knochen eingearbeitet werden, so dass der Operateur die Lage der Anlagefläche vor der Bearbeitung des Knochens beliebig verändern kann, um die optimale Anpassung zu erreichen. Dies gilt auch hinsichtlich der Auswahl von Implantaten mit geeigneten geometrischen Daten, durch Auswahl von Implantaten mit verschiedenen geometrischen Daten und entsprechend angepasster Lage der Anlagefläche kann ebenfalls der Winkel zwischen Femur und Tibia beeinflusst werden.

Günstig ist es, wenn man die Lage der unveränderten Gelenkfläche durch Lagebestimmung mindestens eines ausgewählten Punktes der unveränderten Gelenkfläche bestimmt. Es ist möglich, die unveränderte Gelenkfläche durch nur einen Punkt zu bestimmen, beispielsweise den tiefsten Punkt der Tibiagelenkfläche, es ist aber auch möglich, beispielsweise die Lage mehrerer markanter Punkte dieser Gelenkfläche zu erfassen, um die Lage der unveränderten Gelenkfläche zu bestimmen.

Insbesondere kann man den oder die ausgewählten Punkte der unveränderten Gelenkfläche durch Abtasten bestimmen. Es ist dazu vorteilhaft, wenn die Vorrichtung ein navigiertes Tastinstrument zur Bestimmung der Lage der unveränderten Gelenkfläche aufweist.

Vorzugsweise definiert man die Lage der Anlagefläche vor der Zubereitung der Anlagefläche mittels eines neben Tibia und/oder Femur angeordneten Instrumentes. Vorzugsweise bestimmt man die Lage des Instrumentes mittels eines Navigationssystems, so dass man indirekt durch das Instrument auch die Lage der Anlagefläche durch das Navigationssystem bestimmt.

Es ist günstig, wenn man als Instrument eine Sägelehre verwendet, die dann gleich zur Führung einer Säge verwendet wird, mit der die Anlagefläche in den Knochen eingearbeitet wird.

Den Winkel zwischen Femur und Tibia kann man vorzugsweise auf einem Bildschirm anzeigen, so dass der Operateur sofort sieht, welche Einflüsse eine Lageänderung der Anlagefläche und damit des unikondylären Implantates auf den Winkel zwischen Femur und Tibia hat. Der Operateur kann beispielsweise die Sägelehre neben dem zu bearbeitenden Knochen bewegen, bis der Winkel zwischen Femur und Tibia die gewünschte Größe einnimmt, und dann kann diese Sägelehre in der erreichten Position relativ zu dem Knochen fixiert werden, so dass bei Durchführung des Sägeschnittes dann die dem gewünschten Winkel zwischen Femur und Tibia entsprechende Lage der Anlagefläche sichergestellt ist.

Die nachfolgende Beschreibung bevorzugter Ausführungsformen der Erfindung dient im Zusammenhang mit der Zeichnung der näheren Erläuterung. Es zeigen:
- Figur 1:: eine schematische Ansicht einer Vorrichtung zur Bestimmung des Winkels zwischen Femur und Tibia mit einem Navigationssystem, einer Datenverarbeitungseinrichtung und einem navigierten Instrument zur Definition der Anlagefläche eines unikondylären Implantates und
- Figur 2:: eine schematische Ansicht des proximalen Tibiaendes mit einer unveränderten Gelenkfläche und einer durch ein unikondyläres Implantat ersetzten Gelenkfläche.

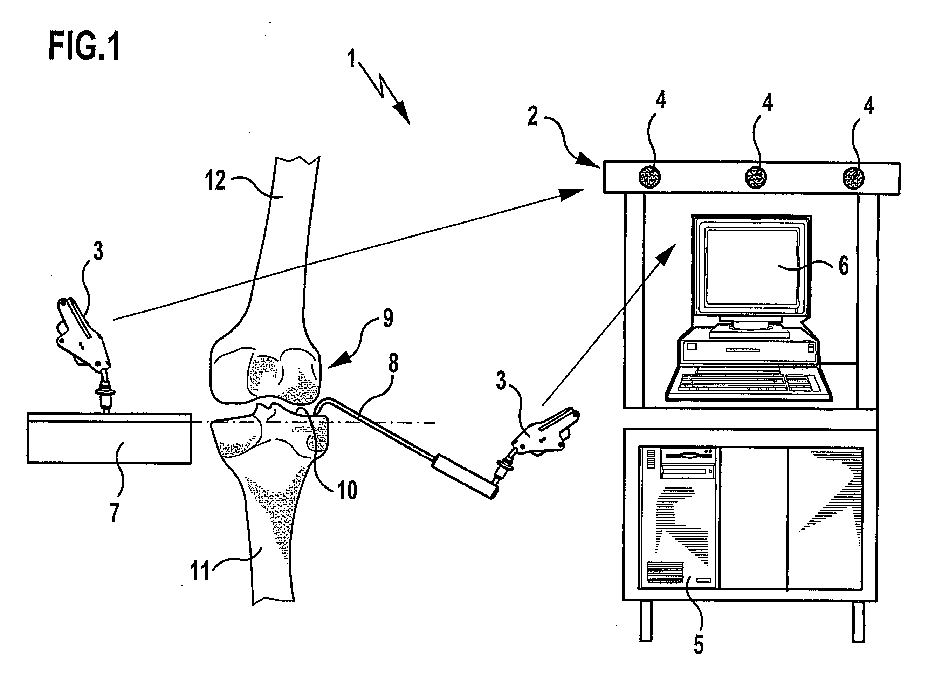

Die in Figur 1 dargestellte Vorrichtung 1 umfasst ein Navigationssystem 2 an sich bekannter Art, mit dem die Lage von Markierelementen 3, also die Position und die Orientierung, festgestellt werden kann. Diese Markierelemente 3 können an Instrumenten und anderen Gegenständen starr befestigt sein, so dass damit auch die Lage dieser Instrumente im Raum feststellbar ist.

Derartige Navigationssysteme 2 arbeiten z.B. mit Hilfe von Infrarotstrahlung, die von mehreren, im Abstand zueinander angeordneten Sende- und Empfangseinrichtungen 4 ausgesandt, an unterschiedlichen Stellen des Markierelementes 3 reflektiert und dann von den Sende- und Empfangseinrichtungen 4 wieder empfangen wird, die daraus gewonnenen Lagedaten des Markierelementes 3 und damit des Gegenstandes, an dem das Markierelement 3 befestigt ist, werden von dem Navigationssystem 2 einer Datenverarbeitungseinrichtung 5 zugeführt, der ein Bildschirm 6 zugeordnet ist.

Die beschriebene Vorrichtung 1 umfasst weiterhin eine Sägelehre 7, die in der Zeichnung nur sehr schematisch dargestellt ist und die dazu dient, ein oszillierendes Sägeblatt einer Knochensäge in einer Ebene zu führen, sowie ein Tastinstrument 8, mit dessen Spitze ausgewählte Punkte an Knochenstrukturen angefahren werden können. Sowohl die Sägelehre 7 als auch das Tastinstrument 8 sind jeweils mit einem Markierelement 3 verbunden, so dass die Lage von Sägelehre und Tastinstrument durch das Navigationssystem 2 kontinuierlich erfassbar ist.

Die beschriebene Vorrichtung 1 dient der Vorbereitung eines Kniegelenks 9 zur Implantation einer unikondylären Knieendoprothese. Bei dieser Operation bleibt eine der beiden Gelenkflächen 10 unverändert, während die andere Gelenkfläche entfernt und sowohl an der Tibia 11 als auch am Femur 12 durch ein unikondyläres Implantat 13 ersetzt wird.

Zur Vorbereitung der Operation wird zunächst in an sich bekannter Weise sowohl für die Tibia 11 als auch für den Femur 12 eine Längsachse bestimmt. Dies lässt sich beispielsweise dadurch erreichen, dass für beiden Knochen der Mittelpunkt des Kniegelenks und der Mittelpunkt des Hüftgelenks bzw. des Knöchelgelenks bestimmt wird, diese Punkte werden zur Festlegung der Längsachsen verwendet. Bei dieser Bestimmung werden sowohl Femur als auch Tibia mit weiteren Markierelementen versehen, die in der Zeichnung nicht dargestellt sind. Diese Markierelemente dienen dann auch dazu, die Lage von Femur und Tibia über das Navigationssystem zu bestimmen.

Mit Hilfe des Tastinstruments 8 werden geometrische Daten der unveränderten Gelenkfläche 10 aufgenommen, dazu wird mit dem Tastinstrument 8 entweder nur ein ausgewählter Punkt angefahren, gegebenenfalls auch mehrere ausgewählte Punkte, deren Lagedaten in der Datenverarbeitungseinrichtung 5 gespeichert werden.

Die Sägelehre 7 wird neben dem zu bearbeitenden Knochen angeordnet und so orientiert, dass die von ihr definierte Sägeebene eine Anlagefläche 14 für das Implantat 13 definiert. Diese Anlagefläche 14 wird in der Regel in derselben Ebene liegen, in der die Sägelehre 7 das Sägeblatt einer Säge führt, die die Anlagefläche 14 definierende Ebene liegt dann neben der Sägelehre 7. Die Lagedaten für eine bestimmte Position der Sägelehre und damit für eine bestimmte angenommene Anlagefläche 14 werden ebenfalls der Datenverarbeitungseinrichtung 5 zugeführt und in dieser gespeichert.

Schließlich werden in dieser Datenverarbeitungseinrichtung 5 noch die geometrischen Daten des verwendeten Implantats 13 gespeichert, beispielsweise die Höhe des Implantates.

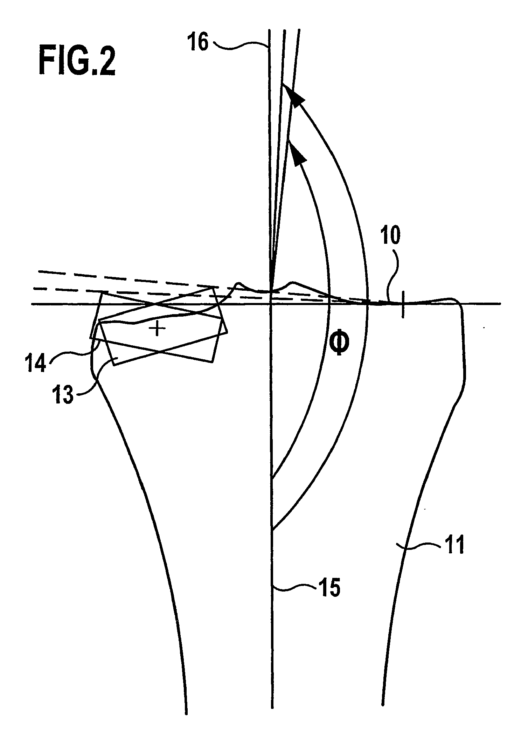

Aus diesen in der Datenverarbeitungseinrichtung 5 gespeicherten Daten berechnet diese den Winkel zwischen den Längsachsen von Tibia und Femur, dieser Winkel hängt von den geometrischen Daten des Implantates und von der Lage des Implantates im Knochen ab, sowohl eine axiale Verschiebung in Richtung der Längsachse der Knochen als auch eine Verschwenkung führt zu einer unterschiedlichen Positionierung des Implantates relativ zur unveränderten Gelenkfläche 10 und damit zu einer Veränderung des Winkels Φ zwischen der Längsachse 15 des Femurs 12 und der Längsachse 16 der Tibia 11. In Figur 2 sind verschiedene derartige Winkel dargestellt, die sich aufgrund der unterschiedlichen Positionierung des Implantates 13 ergeben können. Dabei geht natürlich auch ein, wie das Implantat am jeweils anderen Knochen angeordnet ist, und daher wird hinsichtlich der Anordnung des Implantates am anderen Knochen entweder von einer bestimmten Position ausgegangen, die angenommen wird, oder es wird an beiden Knochen in ähnlicher Weise eine Lage der Anlagefläche angenommen und mittels einer Sägelehre 7 so variiert, bis die gewünschte Winkelorientierung der Längsachsen 15 und 16 erreicht wird.

Die Datenverarbeitungseinrichtung 5 übermittelt ein schematisches Bild des Kniegelenkes an den Bildschirm 6 und stellt auf diesem den Winkel dar, der sich zwischen der Längsachse 15 und der Längsachse 16 einstellt, gegebenenfalls können auf dem Bildschirm zwei um 90° verdrehte Ansichten dargestellt werden, so dass die Abwinkelung in unterschiedlichen Richtungen sichtbar wird. Die Darstellung auf dem Bildschirm kann etwa der Darstellung der Figur 2 entsprechen, in der schematisch nebeneinander die unveränderte Gelenkfläche sowie die Lage und Orientierung des Implantates 13 entsprechend der angenommenen Lage der Anlagefläche 14 sichtbar werden. Zusätzlich werden die Längsachsen 15 und 16 und der von ihnen eingeschlossene Winkel Φ dargestellt.

Wenn der Operateur die Lage der Sägelehre 7 relativ zum Knochen verändert, bedeutet dies, dass auch die angenommene Lage der Anlagefläche 14 verschoben wird, und dies führt unmittelbar zu einer Veränderung des Winkels Φ zwischen den beiden Längsachsen 15 und 16. Der Operateur kann durch Lageveränderung der Anlagefläche 14 somit die gewünschte Orientierung dieser Längsachsen einstellen und somit die Anlagefläche 14 bestimmen, die für ein bestimmtes Implantat 13 in den Knochen eingearbeitet werden muss.

## Patentansprüche

1. Vorrichtung zur Bestimmung des Winkels zwischen Femur (12) und Tibia (11) bei der Implantation einer unikondylären Knieprothese mit einem Navigationssystem (2), einer Datenverarbeitungseinrichtung (5), einem Instrument (7) zur Definition der Lage einer Anlagefläche (14) für ein unikondyläres Implantat (13) an der Tibia (11) und/oder am Femur (12), dessen Lage relativ zu der Lage der unveränderten Gelenkfläche (10) durch das Navigationssystem (2) bestimmbar ist, mit einem navigierten Tastinstrument (8) zur Bestimmung der Lage der unveränderten Gelenkfläche (10) und mid einem Speicher in der Datenverarbeitungseinrichtung (5), in dem die Lagedaten des oder der von dem Tastinstrument (8) angefahrenen Punkte der unveränderten Gelenkfläche (10) gespeichert werden, wobei die Datenverarbeitungseinrichtung (5) so programmiert ist, dass sie unter Verwendung der Lagedaten der unverändert bleibenden Gelenkfläche und der angenommenen Lage der Anlagefläche sowie der geometrischen Daten des unikondylären Implantates (13) den Winkel (Φ) zwischen Femur (12) und Tibia (11) berechnet.

2. Vorrichtung nach Anspruch 1, **dadurch gekennzeichnet, dass** das Instrument (7) eine Sägelehre ist.

3. Vorrichtung nach einem der Ansprüche 1 oder 2, **dadurch gekennzeichnet, dass** sie einen Bildschirm (6) umfasst, auf dem die von der Datenverarbeitungseinrichtung (5) berechneten Winkel (Φ) zwischen Femur (12) und Tibia (11) angezeigt werden.

## Claims

1. An apparatus for determining the angle between a femur (12) and a tibia (11) in the implantation of a unicondylar knee prosthesis, comprising a navigation system (2), a data processing system (5), an instrument (7) for defining the position of a contact surface (14) for a unicondylar implant (13) on the tibia (11) and/or on the femur (12), the position of which relative to the position of the unchanged joint surface (10) may be determined by means of the navigation system (2), a navigated sensing instrument (8) for determining the position of the unchanged joint surface (10), and a memory in the data processing system (5) in which the position data of the point or points, encountered by the sensing instrument (8), of the unchanged joint surface (10) are stored, the data processing system (5) being programmed so that, using the position data of the joint surface remaining unchanged and the assumed position of the contact surface as well as the geometric data of the unicondylar implant (13), it calculates the angle (Φ) between the femur (12) and the tibia (11).

2. An apparatus according to Claim 1, **characterised in that** the instrument (7) is a saw gauge.

3. An apparatus according to one of Claims 1 or 2, **characterised in that** it comprises a display screen (6) on which the angles (Φ) between the femur (12) and the tibia (11), calculated by the data processing system (5), are displayed.

## Revendications

1. Dispositif pour la détermination de l'angle entre le fémur (12) et le tibia (11) lors de l'implantation d'une prothèse unicondylaire du genou avec un système de navigation (2), un dispositif de traitement de données (5), un instrument (7) pour la définition de la position d'une surface d'appui (14) pour un implant unicondylaire (13) dans le tibia (11) et/ou dans le fémur (12) dont la position par rapport à la position de la surface d'articulation inchangée (10) peut être déterminée par le système de navigation (2), avec un instrument à palpeur navigué (8) pour la détermination de la position de la surface d'articulation inchangée (10) et avec une mémoire dans le dispositif de traitement de données (5) dans laquelle les données relatives à la position du point ou des points de la surface d'articulation inchangée (10) parcouru ou parcourus par l'instrument à palpeur (8) sont mémorisées, dans lequel le dispositif de traitement de données (5) est programmé de telle manière qu'il calcule l'angle (Φ) entre le fémur (12) et le tibia (11) en se servant des données relatives à la position de la surface d'articulation restant inchangée et de la position prise par la surface d'appui ainsi que des données géométriques de l'implant unicondylaire (13).

2. Dispositif selon la revendication 1, **caractérisé en ce que** l'instrument (7) est un gabarit de scie.

3. Dispositif selon l'une quelconque des revendications 1 ou 2, **caractérisé en ce qu'**il comporte un écran (6) sur lequel les angles (Φ) entre le fémur (12) et le tibia (11) calculés par le dispositif de traitement de données (5) sont affichés.
